# EUROPEAN PATENT APPLICATION

(11) **EP 4 830 617 A1**
(43) Date of publication of application: **28.10.2026**
(21) Application number: 24907776.9
(22) Date of filing: 06.11.2024
(51) Int. Cl.: A61B 5/00, A61B 5/0245, A61B 5/024, A61B 5/01, A61B 5/1455, A61B 5/021

(54) **SLEEP ANALYSIS SYSTEM CONSIDERING CIRCADIAN RHYTHM AND AMOUNT OF SUNSHINE AND SLEEP ANALYSIS METHOD USING SAME**

(30) Priority: 19.12.2023 KR 20230185645
(71) Applicant: Osong Medical Innovation Foundation, Cheongju-si, Chungcheongbuk-do 28160 (KR)
(72) Inventor: LEE, Seungrag, Sejong 30101 (KR); LEE, Kiri, Sejong 30123 (KR); KANG, Shinil, Pyeongtaek-si Gyeonggi-do 18024 (KR); LEE, Minsuk, Cheongju-si Chungcheongbuk-do 28165 (KR); KIM, Hyunjun, Gwangmyeong-si Gyeonggi-do 14349 (KR); BANG, Hyeonjin, Cheongju-si Chungcheongbuk-do 28673 (KR); MOON, Dongjun, Sejong 30098 (KR)
(74) Representative: Lewis Silkin LLP
(86) International application number: PCT/KR2024/017436
(87) International publication number: WO 2025/135491

(57) **Abstract**

In a sleep analysis system considering circadian rhythm and daylight exposure, and a sleep analysis method, the sleep analysis system includes a data generation unit, a data processing unit, and a data output unit. The data generation unit measures data for sleep analysis and includes an illuminance measurement unit configured to selectively measure overall light or light of a specific wavelength. The data processing unit processes the measured data and includes a light amount calculation unit configured to selectively calculate an overall light amount of the light or a light amount of the specific wavelength based on measurement results of the illuminance measurement unit. The data output unit displays a circadian rhythm and a sleep state to the outside based on the processed data.

## Description

### BACKGROUND OF THE INVENTION

### TECHNICAL FIELD

The present invention relates to a sleep analysis system considering circadian rhythm and daylight exposure and a sleep analysis method using the same, and more particularly, to a sleep analysis system considering circadian rhythm and daylight exposure, and a sleep analysis method using the same, capable of quantitatively measuring a wearer's circadian rhythm based on illuminance and biometric signal information and analyzing and monitoring a sleep state of the wearer.

### DISCUSSION OF THE RELATED ART

Circadian rhythm refers to all physiological phenomena having a periodicity of 24 hours, and is influenced by endogenous factors and exogenous factors. The endogenous factors are controlled by a genetic time-regulating system, such that physiological functions including body temperature, immunity, and hormones exhibit periodicity, while the exogenous factors act on circadian rhythm through environmental elements and individual behavioral patterns.

In particular, when a problem occurs in the circadian rhythm, disorders such as emotional disorders, sleep disorders, depression, and fatigue may occur, and in order to treat such disorders, a measurement device capable of quantitatively measuring the circadian rhythm is required.

Accordingly, Korean Patent No. 10-2545257 discloses a technology related to a sleep management system using a wearable device, and Korean Patent No. 10-2417541 discloses a technology related to feedback-based circadian rhythm management.

However, in the conventional sleep management technology based on measurement of circadian rhythm, only an intensity of externally exposed light is measured using a single photodiode, which is limited in accurately measuring the circadian rhythm, and thus has a problem in that accuracy of sleep analysis is not high.

Related prior art documents include Korean Patent No. 10-2545257 and Korean Patent No. 10-2417541.

### SUMMARY

Accordingly, the technical problem of the present invention has been devised in view of the above circumstances, and an object of the present invention is to provide a sleep analysis system considering circadian rhythm and daylight exposure, capable of quantitatively measuring a wearer's circadian rhythm based on illuminance and biometric signal information and analyzing and monitoring a sleep state of the wearer.

Another object of the present invention is to provide a sleep analysis method using the sleep analysis system.

According to one aspect of the present invention, a sleep analysis system includes a data generation unit, a data processing unit, and a data output unit. The data generation unit measures data for sleep analysis and includes an illuminance measurement unit configured to selectively measure overall light or light of a specific wavelength. The data processing unit processes the measured data and includes a light amount calculation unit configured to selectively calculate an overall light amount of the light or a light amount of the specific wavelength based on measurement results of the illuminance measurement unit. The data output unit displays a circadian rhythm and a sleep state to the outside based on the processed data.

In an example embodiment, the illuminance measurement unit may include a lens through which light is incident, a transmissive grating spaced apart from the lens by a predetermined distance and configured to disperse the incident light into visible light wavelengths, and a camera sensor spaced apart from the transmissive grating by a predetermined distance d and configured to measure an intensity of light of the visible light wavelengths.

In an example embodiment, the camera sensor may measure an intensity of light over an entire visible light wavelength range, or may measure an intensity of light for each wavelength of the visible light by varying the distance d from the transmissive grating.

In an example embodiment, the light amount calculation unit may include a light amount measurement unit configured to obtain a light amount over an entire visible light wavelength range from an intensity of light measured by the camera sensor, a pixel-based light amount collection unit configured to collect light amount information for each pixel unit of the camera sensor, a wavelength conversion unit configured to convert pixel units into wavelength units, a first light amount calculation unit configured to calculate a light amount over the entire visible light wavelength range from the light amount information converted into the wavelength units, and a second light amount calculation unit configured to calculate a light amount at a specific wavelength from the light amount information converted into the wavelength units.

In an example embodiment, the second light amount calculation unit may calculate a light amount in a blue light wavelength range as the specific wavelength.

In an example embodiment, the illuminance measurement unit may include a light sensor configured to measure an overall intensity of light, or may include N light sensors (N being a natural number) each configured to measure an intensity of light of a specific wavelength, and the light amount calculation unit may obtain a light amount over an entire wavelength range of the light from the overall intensity of light, or obtain light amounts at each of N wavelengths.

In an example embodiment, the data generation unit may include an electrocardiogram measurement unit configured to measure a user's electrocardiogram, a photoplethysmography measurement unit configured to measure a user's photoplethysmography in synchronization with the electrocardiogram measurement, and a temperature measurement unit configured to measure a user's body temperature in synchronization with the electrocardiogram measurement.

In an example embodiment, the data processing unit may include a photoplethysmography calculation unit configured to derive oxygen saturation and blood pressure of the user from photoplethysmography measurement results, an electrocardiogram calculation unit configured to derive a heart rate and heart rate variability of the user from electrocardiogram measurement results, and a body temperature calculation unit configured to derive body temperature of the user from body temperature measurement results.

In an example embodiment, the data output unit may display information derived by the data processing unit to the outside according to time of a circadian cycle.

In an example embodiment, the data output unit may display a state of the user during sleep time among the information derived by the data processing unit.

In an example embodiment, the data output unit may selectively display a change in overall daylight exposure during sleep time calculated by the light amount calculation unit and a change in a light amount of a specific wavelength.

In an example embodiment, the data generation unit, the data processing unit, and the data output unit may be mounted in a wearable device.

According to another aspect of the present invention, a sleep analysis method includes selectively measuring overall light or light of a specific wavelength, selectively calculating an overall light amount or a light amount of the specific wavelength from the measured light, displaying an alarm and measuring a user's electrocardiogram, generating a synchronization signal synchronized with the electrocardiogram measurement, measuring a user's photoplethysmography and a user's body temperature based on the synchronization signal, calculating oxygen saturation, heart rate, blood pressure, heart rate variability, and body temperature of the user from the synchronized electrocardiogram, photoplethysmography, and body temperature measurements, and displaying a circadian rhythm and a sleep state to the outside based on the calculated information.

According to exemplary embodiments of the present invention, analysis results of a user's circadian rhythm and sleep state may be checked through a wearable device, and in particular, in analysis of the circadian rhythm or the sleep state, in addition to analysis based simply on an overall amount of light, an amount of light of a specific wavelength that has a significant influence on the sleep state, for example, blue light, may be individually analyzed, thereby further improving accuracy and reliability of derived analysis results.

In this case, the wavelength of the light of the specific wavelength may be variously selected, and information on light of a specific wavelength other than blue light may be selectively provided, such that more diverse circadian rhythm analysis results and sleep analysis results may be provided.

In particular, in order to obtain amounts of light for each of various wavelengths, a photodiode corresponding to each wavelength and an optical filter for each wavelength are generally required; however, according to the present example embodiment, amounts of light for various wavelengths may be obtained through a single illuminance measurement unit, thereby enabling a simplified system design and facilitating implementation as a wearable device.

That is, the illuminance measurement unit may measure an intensity of light for each of various visible light wavelengths through a transmissive grating configured to disperse light and a camera sensor having a separation distance from the transmissive grating that is variable, and thus measurement of the intensity of light for each wavelength is possible while omitting a plurality of photodiodes or optical filters.

Furthermore, as various sensors are provided and sensing results are synchronized with each other and analyzed over time, not only analysis of the user's circadian rhythm but also separate analysis of sleep time is possible, and through this, more accurate and reliable sleep analysis results may be provided.

### BRIEF DESCRIPTION OF THE DRAWINGS

FIG. 1 is a block diagram illustrating a sleep analysis system according to an example embodiment of the present invention;
FIG. 2A and FIG. 2B are a plan view and a rear view illustrating an example in which the sleep analysis system of FIG. 1 is implemented as a wearable device;
FIG. 3 is a flowchart illustrating a sleep analysis method using the sleep analysis system of FIG. 1;
FIG. 4A and FIG. 4B are schematic views illustrating an illuminance measurement step through the illuminance measurement unit of FIG. 1 and a light amount calculation step of a light amount calculation unit, respectively;
FIG. 5 is a block diagram illustrating the light amount calculation unit of FIG. 1;
FIG. 6 is a flowchart illustrating in detail a step of measuring illuminance through the illuminance measurement unit of FIG. 1;
FIG. 7 is views exemplarily illustrating a state in which circadian rhythm monitoring results are displayed through the wearable device of FIG. 2A; and
FIG. 8 is views exemplarily illustrating a state in which sleep state analysis results are displayed through the wearable device of FIG. 2A.

### <Reference numerals>

| | | | |
|---|---|---|---|
| 10 : | sleep analysis system | 20 : | wearable device |
| 100 : | data generation unit | 140 : | illuminance measurement unit |
| 142: | lens | 143 : | transmissive grating |
| 144 : | camera sensor | 200 : | data processing unit |
| 260 : | light amount calculation unit | 261 : | light amount measurement unit |
| 262 : | pixel-based light amount collection unit | 263 : | wavelength conversion unit |
| 264 : | first light amount calculation unit | 265 : | second light amount calculation unit |
| 300 : | data output unit | | |

### DETAILED DESCRIPTION OF THE INVENTION

The present invention may be variously modified and may have various forms, and example embodiments thereof will be described in detail in the following description. However, this is not intended to limit the present invention to specific disclosed forms, and it should be understood that the present invention includes all modifications, equivalents, and alternatives falling within the spirit and scope of the present invention. In describing the drawings, like reference numerals are used for like components.

Terms such as "first" and "second" may be used to describe various components, but such components should not be limited by these terms. These terms are only used to distinguish one component from another. Terms used herein are for the purpose of describing particular example embodiments only and are not intended to limit the present invention. Singular expressions include plural expressions unless the context clearly indicates otherwise.

In this application, terms such as "include" or "comprise" are intended to specify the presence of stated features, numbers, steps, operations, components, parts, or combinations thereof, but do not preclude the presence or addition of one or more other features, numbers, steps, operations, components, parts, or combinations thereof.

Unless otherwise defined, all terms including technical and scientific terms used herein have the same meaning as commonly understood by one of ordinary skill in the art to which the present invention belongs. Terms defined in generally used dictionaries should be interpreted as having meanings consistent with their meanings in the context of the relevant art and should not be interpreted in an idealized or overly formal sense unless expressly defined herein.

Hereinafter, the present invention will be explained in detail with reference to the accompanying drawings.

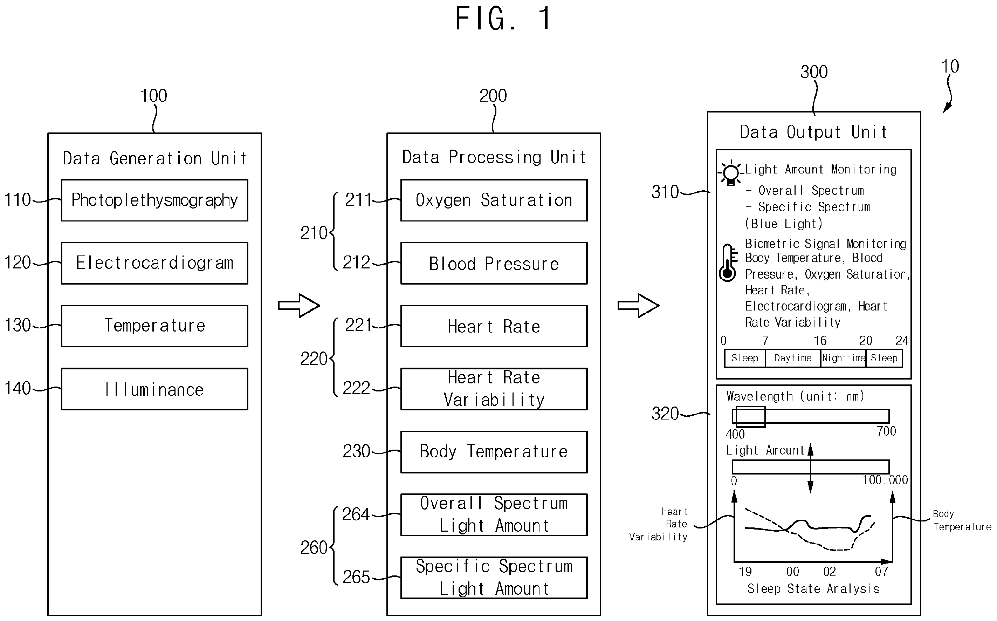

FIG. 1 is a block diagram illustrating a sleep analysis system according to an example embodiment of the present invention.

Referring to FIG. 1, the sleep analysis system 10 according to the present example embodiment includes a data generation unit 100, a data processing unit 200, and a data output unit 300.

The data generation unit 100 measures data for analysis of a circadian rhythm and a sleep state, and specifically includes a photoplethysmography measurement unit 110, an electrocardiogram measurement unit 120, a temperature measurement unit 130, and an illuminance measurement unit 140.

First, the electrocardiogram measurement unit 120 measures electrocardiogram information of a user, and although not illustrated, may include an alarm configured to notify an outside that electrocardiogram measurement has started.

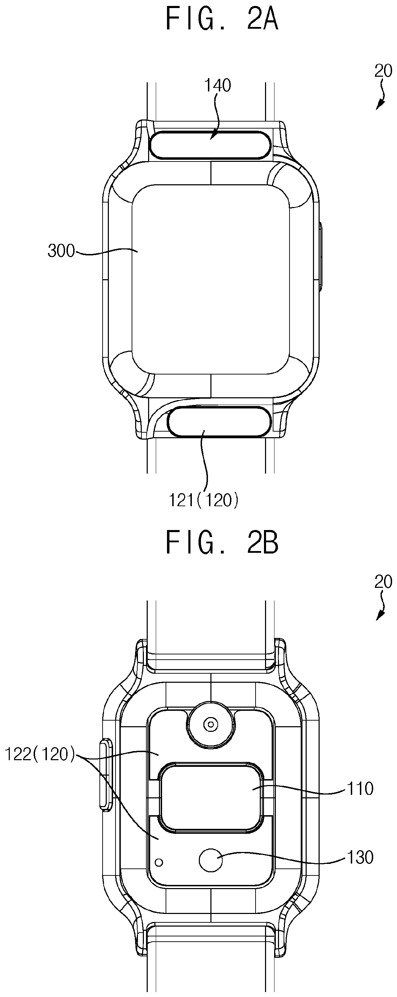

The electrocardiogram measurement unit 120 may be variously configured, and as illustrated in FIG. 2A and FIG. 2B, may include a pair of electrodes to measure an electrocardiogram signal of the user.

The photoplethysmography measurement unit 110 measures photoplethysmography of the user, and the temperature measurement unit 130 measures a body temperature of the user. In this case, the photoplethysmography and the body temperature should be measured in synchronization with the electrocardiogram measurement, that is, synchronized with each other over time.

For this purpose, the electrocardiogram measurement unit 120 may generate a notification indicating measurement of an electrocardiogram signal and simultaneously provide a synchronization signal to the photoplethysmography measurement unit 110 and the temperature measurement unit 130, and upon receiving the synchronization signal, the photoplethysmography and the body temperature are measured.

The illuminance measurement unit 140 measures an intensity of light provided from outside, and may measure an overall intensity of light or selectively measure an intensity of light for a specific wavelength.

That is, in the present example embodiment, as will be described later, the illuminance measurement unit 140 may integrally measure an overall intensity of incident light, and alternatively, may selectively and individually measure only an intensity of light corresponding to a specific wavelength.

Furthermore, the measurement of the overall intensity of light or the measurement of the intensity of light for the specific wavelength may be selectively performed, or both may be performed simultaneously, and this may be performed according to a user's selection or a separate control command.

Furthermore, the data generation unit 100 may measure data other than those measured by the measurement units illustrated in FIG. 1, and in order to measure other data, may be configured to additionally or separately include a measurement unit for measuring the corresponding data.

In addition, signals measured by the respective measurement units 110, 120, 130, and 140 of the data generation unit 100 are provided to the data processing unit 200.

Accordingly, the data processing unit 200 performs data processing such as predetermined calculations based on the signals measured by the respective measurement units of the data generation unit 100, and derives basic information for obtaining circadian rhythm information and sleep state information.

That is, the data processing unit 200 may include an oxygen saturation calculation unit 210, an electrocardiogram calculation unit 220, a body temperature calculation unit 230, and a light amount calculation unit 260.

First, the oxygen saturation calculation unit 210 derives oxygen saturation information 211 and blood pressure information 212 of the user based on the photoplethysmography of the user measured by the photoplethysmography measurement unit 110.

The electrocardiogram calculation unit 220 derives heart rate information 221 and heart rate variability information 222 of the user based on the electrocardiogram of the user measured by the electrocardiogram measurement unit 120.

The body temperature calculation unit 230 derives body temperature information 230 of the user based on the body temperature of the user measured by the temperature measurement unit 130.

The light amount calculation unit 260 selectively derives an overall light amount 264 of incident light or a light amount 265 of a specific wavelength based on intensity information of incident light measured by the illuminance measurement unit 140. In this case, a specific method for deriving the light amount in the light amount calculation unit 260 will be described later in detail.

As described above, through the data processing unit 200, required data are derived by performing data processing or calculations based on information measured by the data generation unit 100, and the derived data are provided to the data output unit 300.

Accordingly, the data output unit 300 outputs the processed data to the outside, and may output information related to the user's circadian rhythm through a circadian rhythm output unit 310 or output analysis results of the user's sleep state through a sleep state output unit 320.

At this time, specific types and contents of information output through the data output unit 300 will be described later with reference to the drawings.

FIG. 2A and FIG. 2B are a plan view and a rear view illustrating an example in which the sleep analysis system of FIG. 1 is implemented as a wearable device.

The sleep analysis system 10 described with reference to FIG. 1 may be implemented and manufactured as a wearable device 20, as illustrated in FIG. 2A and FIG. 2B.

In particular, the wearable device 20 may be a band-type watch, and while being worn on a wrist of a user, may perform analysis of a circadian rhythm and a sleep state of the user.

Referring to FIG. 2A and FIG. 2B, when the sleep analysis system 10 is implemented in the wearable device 20, the data output unit 300 is provided on a front surface of the device 20 in a display form.

In addition, since the illuminance measurement unit 140 needs to measure external light, the illuminance measurement unit 140 is provided on the front surface of the device 20, similarly to the data output unit 300, and is formed to face outward. For example, the illuminance measurement unit 140 may be provided on an upper outer portion of the data output unit 300.

Further, in the electrocardiogram measurement unit 120, a first electrode 121 may be provided on the front surface of the device 20 on a lower outer portion of the data output unit 300, and a second electrode 122 may be entirely provided on a rear surface of the device 20. That is, the second electrode 122 is provided on the rear surface of the device 20 and is formed to be in direct contact with the user's skin.

In addition, the photoplethysmography measurement unit 110 is also formed on the rear surface of the device 20 to be in contact with the user's skin, and although the drawings exemplarily illustrate that the photoplethysmography measurement unit 110 is formed at a central portion, the present invention is not limited thereto, and it is sufficient that the photoplethysmography measurement unit 110 is in direct contact with the user's skin.

In addition, the temperature measurement unit 130 is formed on the rear surface of the device 20, and although the drawings exemplarily illustrate that the temperature measurement unit 130 is formed at a lower portion of one side, the present invention is not limited thereto, and both non-contact type and contact type are possible, and the temperature measurement unit 130 may be mounted at various positions.

As described above, the data generation unit 100 and the data output unit 300 are provided on the front surface or the rear surface of the wearable device 20 to be exposed to the outside, whereas the data processing unit 200 performs calculations on the measured data and thus may be provided inside a main body of the wearable device 20.

The sleep analysis system 10 described with reference to FIG. 1 may be implemented as a band-type wearable device 20 that is wearable on a wrist, as illustrated in FIG. 2A and FIG. 2B; however, the present invention is not limited thereto, and may also be implemented as a wearable device that is worn on other parts of the user's body, for example, a chest or an ankle.

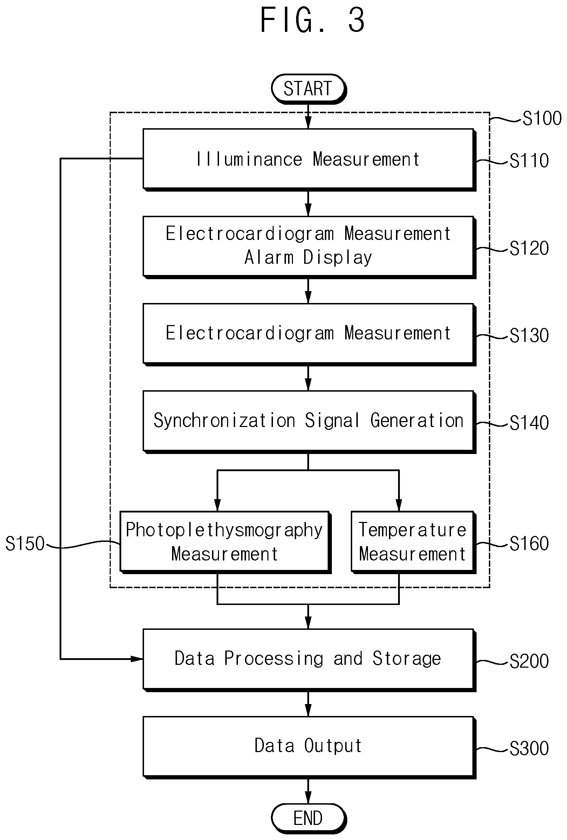

FIG. 3 is a flowchart illustrating a sleep analysis method using the sleep analysis system of FIG. 1.

Referring to FIG. 3, in a sleep analysis method using the sleep analysis system 10 described with reference to FIG. 1, first, illuminance of incident light is measured through the illuminance measurement unit 140 (step S110), and data related to the measured illuminance are processed and stored through the light amount calculation unit 260 (step S200), and then output to the outside through the data output unit 300 (step S300).

In this case, as described above, through the illuminance measurement unit 140, an overall incident light or light of a specific wavelength may be selectively measured, and likewise, through the light amount calculation unit 260, a light amount of an entire spectrum (wavelength range) or a light amount of a specific spectrum may be derived. Furthermore, a more detailed description of the light amount calculation performed by the light amount calculation unit 260 will be provided later with reference to the drawings.

Thereafter, as illustrated in FIG. 3, the electrocardiogram measurement unit 120 notifies an outside of a state in which electrocardiogram measurement is started by displaying a predetermined alarm (step S120), and performs measurement of a user's electrocardiogram (step S130).

In addition, together with the start of electrocardiogram measurement, a synchronization signal for synchronization is generated and provided to the photoplethysmography measurement unit 110 and the temperature measurement unit 130 (step S140), and accordingly, upon receiving the synchronization signal, the photoplethysmography measurement unit 110 and the temperature measurement unit 130 measure the user's photoplethysmography and body temperature, respectively (steps S150 and S160).

That is, the photoplethysmography measurement unit 110, the temperature measurement unit 130, and the electrocardiogram measurement unit 120 are synchronized with each other to measure the user's photoplethysmography, body temperature, and electrocardiogram over time, and through this, the measured data may be synchronized with each other, thereby improving convenience and reliability in final data processing.

Meanwhile, although FIG. 3 illustrates that an electrocardiogram measurement alarm display step (step S120), an electrocardiogram measurement step (step S130), a synchronization signal generation step (step S140), a photoplethysmography measurement step (step S150), and a temperature measurement step (step S160) are sequentially performed, the steps of measuring these respective signals may be substantially performed simultaneously.

Furthermore, the data measured from the user as described above are provided to the data processing unit 200, and the data processing unit 200 derives and stores information such as oxygen saturation, blood pressure, heart rate, heart rate variability, and body temperature of the user from the measured data (step S200).

In this case, detailed derivation of the above information is the same as described with reference to FIG. 1.

Accordingly, the derived information is finally displayed to the outside through the data output unit 300 (step S300).

Hereinafter, detailed contents of illuminance measurement performed by the illuminance measurement unit 140 of FIG. 1 and calculation performed by the light amount calculation unit 260 for calculating a light amount based on the measured illuminance will be described.

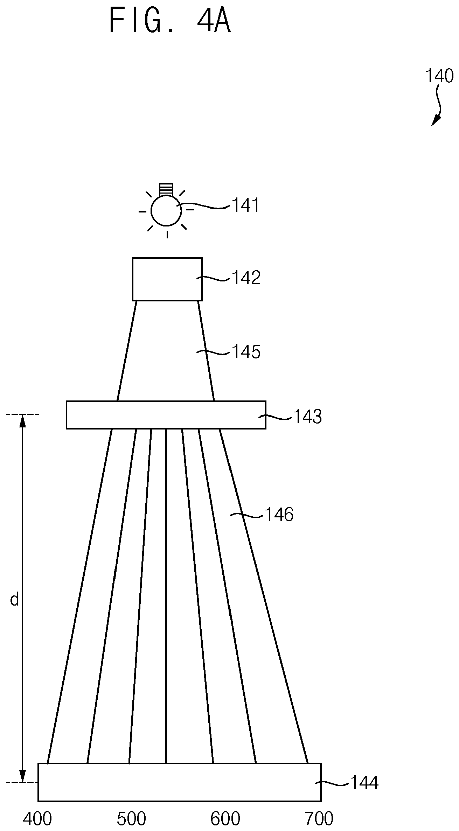

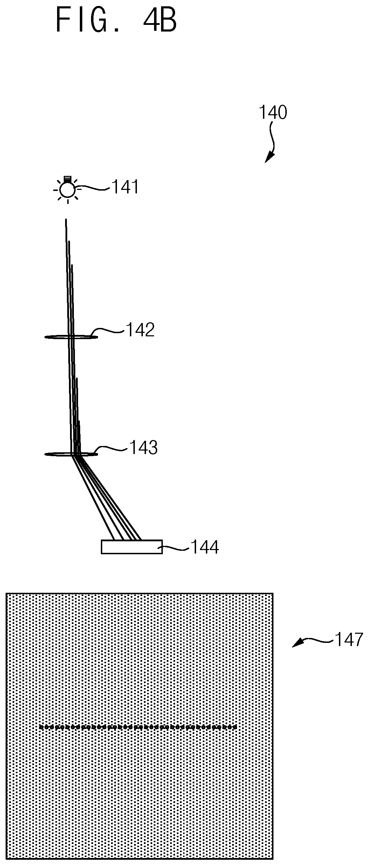

FIG. 4A and FIG. 4B are schematic views illustrating an illuminance measurement step through the illuminance measurement unit of FIG. 1 and a light amount calculation step of a light amount calculation unit, respectively.

First, referring to FIGS. 4A and 4B, the illuminance measurement unit 140 includes a lens 142, a transmissive grating 143, and a camera sensor 144.

The lens 142 transmits light incident from a light source 141, and incident light 145 that has passed through the lens 142 is provided to the transmissive grating 143. In this case, the incident light 145 may be visible light.

The transmissive grating 143 disperses the incident light 145 into a visible light spectrum (wavelength spectrum), and light that has passed through the transmissive grating 143 is provided to the camera sensor 144 as visible light 146 having dispersed wavelengths.

Accordingly, the camera sensor 144 senses the visible light 146 having the dispersed wavelengths, and may measure an intensity of light for each of the dispersed wavelengths of the visible light.

As a result, the incident light 145 that has passed through the lens 142, which may be a slit, is generated as light having a continuous spectrum form by the transmissive grating 143, and when the camera sensor 144 is a line CMOS sensor, an intensity of the spectrum may be measured for each pixel.

That is, in this case, the camera sensor 144 is disposed to be spaced apart from the transmissive grating 143 by a predetermined distance d, and the separation distance d may be variously changed. In general, when the separation distance d is applied to a diffraction grating equation (λ = d × sinθ, where d denotes a grating pitch interval), a pixel distance of the camera sensor 144 may be converted into a wavelength length, and by adjusting a position of the camera sensor 144, that is, the distance d from the transmissive grating 143, an intensity of light in a spectrum within a visible light wavelength range of 400 nm to 700 nm may be measured.

In this case, since pixel information of the camera sensor 144 represents wavelength information, a light amount in a specific wavelength range may be obtained by selecting a range of pixels, which is performed through the light amount calculation unit 260 to be described later.

Alternatively, through the light amount calculation unit 260, N specific wavelength ranges may be designated to obtain light amounts for N wavelengths, which will also be described later through description of the light amount calculation unit 260. In particular, as confirmed through an image 147 of FIG. 4B, a spectrum dispersion effect of white light by the diffraction grating was confirmed using optical simulation software.

As described above, the illuminance measurement unit 140 may measure an intensity of light over an entire visible light wavelength range of incident light (400 nm to 700 nm), and also may individually measure an intensity of light for each wavelength of the visible light by varying the distance d from the transmissive grating 143.

Meanwhile, unlike the above description, although not illustrated, the illuminance measurement unit 140 may be configured with a light sensor (photodiode) configured to measure an overall intensity of light. Accordingly, through the illuminance measurement unit 140, an overall intensity of the entire incident visible light may be measured at once, and through the light amount calculation unit 260 to be described later, a light amount over an entire wavelength range of the incident visible light may be obtained based on the overall intensity of the incident visible light sensed by the light sensor.

In addition, the illuminance measurement unit 140 may be configured with, together with or instead of the light sensor configured to measure the overall intensity of light, N light sensors (N being a natural number) each configured to measure an intensity of light of a specific wavelength.

In this case, each of the N light sensors measures an intensity of light at a specific wavelength to be measured, and accordingly, a number of light sensors may be selectively provided according to a number of specific wavelengths to be measured.

That is, in order to measure only an overall intensity of visible light and an intensity of light in a blue light wavelength range through the illuminance measurement unit 140, only two light sensors (one for an entire wavelength range and one for the blue light wavelength range) may be provided.

Furthermore, when the light sensors of the illuminance measurement unit 140 are provided as described above, the light amount calculation unit 260 obtains light amounts for each of N wavelengths based on intensities of light of the specific wavelengths sensed by the respective N light sensors.

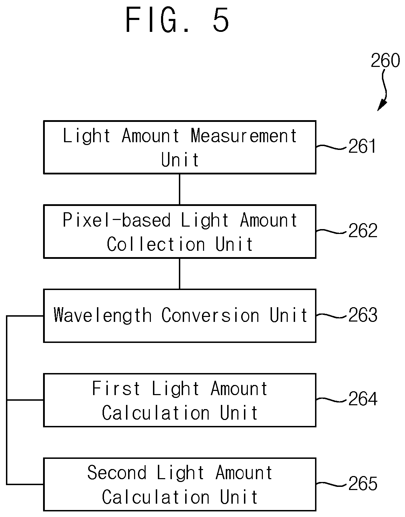

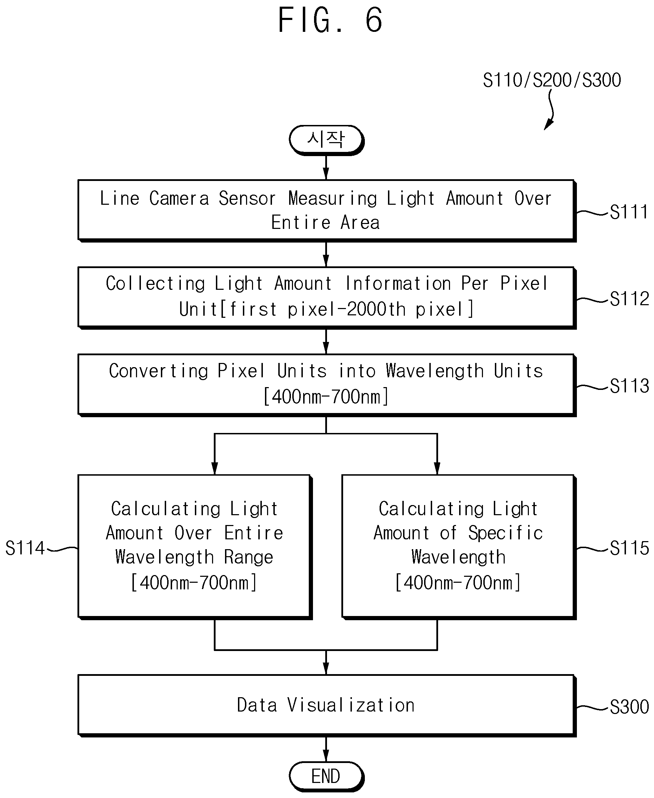

FIG. 5 is a block diagram illustrating the light amount calculation unit of FIG. 1. FIG. 6 is a flowchart illustrating in detail a step of measuring illuminance through the illuminance measurement unit of FIG. 1.

Measurement results related to an intensity of light measured through the illuminance measurement unit 140 described with reference to FIG. 4 are provided to the light amount calculation unit 260. Accordingly, the light amount calculation unit 260 measures illuminance of the light and calculates a light amount (steps S110 and S200).

Specifically, referring to FIG. 5 and FIG. 6, in the step of measuring illuminance of the light and calculating the light amount (steps S110 and S200), first, the light amount measurement unit 261 measures a light amount over an entire area of the camera sensor 144 (step S111). That is, a light amount over an entire visible light wavelength range is obtained from an intensity of light measured by the camera sensor 144.

Thereafter, the pixel-based light amount collection unit 262 collects light amount information for each pixel unit of the camera sensor 144 (step S112). For example, when the camera sensor 144 includes a total of 2,000 pixels, light amount information is collected for each pixel unit from a first pixel to a 2,000th pixel.

Thereafter, the wavelength conversion unit 263 converts the pixel units into wavelength units (step S113). That is, considering a wavelength range of visible light of 400 nm to 700 nm corresponding to the incident light, the pixel units are converted into wavelength units. Such conversion means converting a pixel distance into a wavelength length and selecting pixels of a line CMOS sensor corresponding to a spectrum to measure an intensity of light.

That is, finally, the first light amount calculation unit 264 calculates a light amount over an entire wavelength range of the visible light of 400 nm to 700 nm (step S114), and the second light amount calculation unit 265 calculates a light amount in a specific wavelength range, for example, a blue light wavelength range of 400 nm to 450 nm (step S115).

In this case, since a wavelength range that has the greatest influence on a sleep state is generally a blue light wavelength range, calculation of a light amount in the range of 400 nm to 450 nm is exemplified; however, the wavelength range for the light amount may be variously selected according to a user's selection or necessity.

Thereafter, information on the calculated light amount is displayed to the outside through the data output unit 300 (step S300).

As described above, when measuring daylight exposure, a light amount is calculated based on a total sum of light measured through all pixels of the line CMOS sensor, and when measuring an intensity of light in a specific wavelength range, a pixel distance is converted into a wavelength length to select pixels of the line CMOS sensor corresponding to a spectrum, thereby measuring an intensity of light. Through this, it is possible not only to measure illuminance of entire light and intensities of light in various wavelength bands through a single photo sensor array, but also to select and measure a desired wavelength from an entire spectral wavelength range without replacement of an optical filter or mechanical movement of a spectral slit.

FIG. 7 is views exemplarily illustrating a state in which circadian rhythm monitoring results are displayed through the wearable device of FIG. 2A.

Referring to FIG. 7, through the data output unit 300, results of circadian rhythm monitoring of a user may be displayed as illustrated.

That is, when the user clicks a circadian rhythm measurement button 310, which is a circadian rhythm measurement output unit of the wearable device 20, buttons for light amount, oxygen saturation, blood pressure, and heart rate/body temperature may be generated through the data output unit 300, and by clicking each of the buttons, information on light amount, oxygen saturation, blood pressure, and heart rate/body temperature over time may be displayed and output as illustrated.

In particular, in the case of light amount information, not only light amount values calculated over an entire spectral range but also light amount values calculated in a specific wavelength range may be selectively output either individually or all together.

Through this, results of the user's circadian rhythm over a period of 24 hours may be checked.

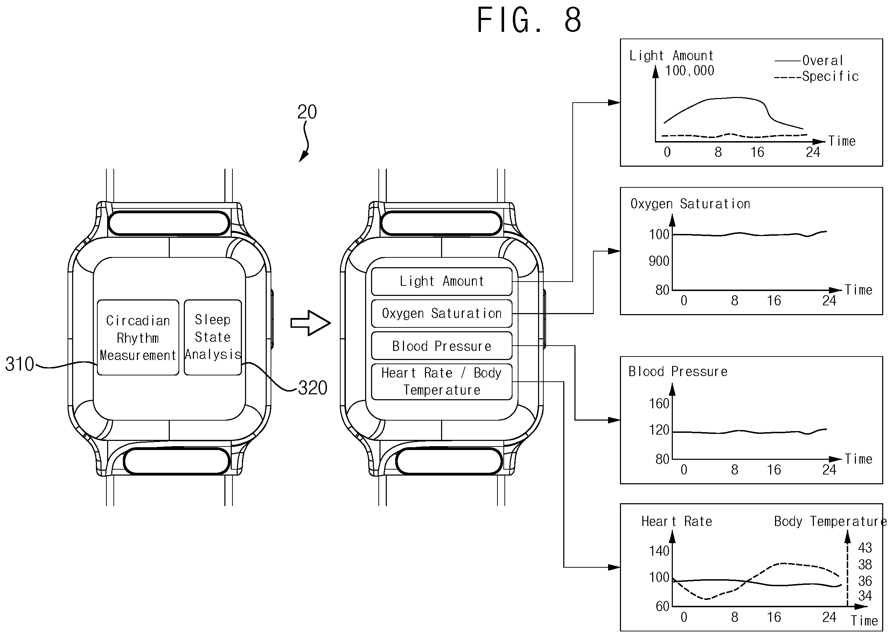

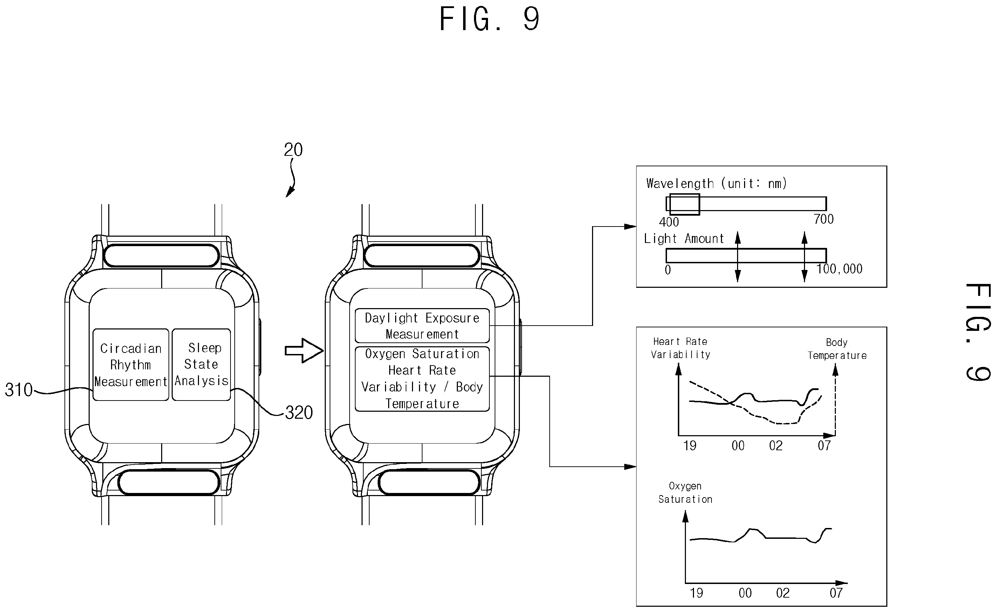

FIG. 8 is views exemplarily illustrating a state in which sleep state analysis results are displayed through the wearable device of FIG. 2A.

Alternatively, referring to FIG. 8, when a user clicks a sleep state analysis button 320, which is a sleep state output unit of the wearable device 20, buttons for daylight exposure measurement and oxygen saturation/heart rate variability/body temperature may be generated through the data output unit 300.

Accordingly, when the user clicks the daylight exposure measurement button, information on a total sum of a light amount calculated over an entire visible light wavelength range and a total sum of a light amount calculated in a specific wavelength range may be selectively or simultaneously output.

In addition, when the user clicks the oxygen saturation/heart rate variability/body temperature button, degrees of change in heart rate variability, body temperature, and oxygen saturation according to nighttime and sleep time may be displayed and output as illustrated.

Accordingly, the user may analyze a sleep rhythm by checking daylight exposure and a light amount of a specific wavelength, for example, blue light, and monitoring heart rate variability, body temperature, and oxygen saturation information according to a change and an amount of the light amount.

In this case, heart rate variability is biometric information related to stress or depression and may be used to analyze factors of sleep disorders, body temperature is biometric information related to melatonin secretion and may be used to obtain information on a sleep state and an appropriate sleep time, and oxygen saturation may be used to detect sleep rhythm disturbance factors such as sleep apnea occurring during sleep.

According to exemplary embodiments of the present invention, analysis results of a user's circadian rhythm and sleep state may be checked through a wearable device, and in particular, in analysis of the circadian rhythm or the sleep state, in addition to analysis based simply on an overall amount of light, an amount of light of a specific wavelength that has a significant influence on the sleep state, for example, blue light, may be individually analyzed, thereby further improving accuracy and reliability of derived analysis results.

In this case, the wavelength of the light of the specific wavelength may be variously selected, and information on light of a specific wavelength other than blue light may be selectively provided, such that more diverse circadian rhythm analysis results and sleep analysis results may be provided.

In particular, in order to obtain amounts of light for each of various wavelengths, a photodiode corresponding to each wavelength and an optical filter for each wavelength are generally required; however, according to the present example embodiment, amounts of light for various wavelengths may be obtained through a single illuminance measurement unit, thereby enabling a simplified system design and facilitating implementation as a wearable device.

That is, the illuminance measurement unit may measure an intensity of light for each of various visible light wavelengths through a transmissive grating configured to disperse light and a camera sensor having a separation distance from the transmissive grating that is variable, and thus measurement of the intensity of light for each wavelength is possible while omitting a plurality of photodiodes or optical filters.

Furthermore, as various sensors are provided and sensing results are synchronized with each other and analyzed over time, not only analysis of the user's circadian rhythm but also separate analysis of sleep time is possible, and through this, more accurate and reliable sleep analysis results may be provided.

Having described exemplary embodiments of the present invention, it is further noted that it is readily apparent to those of reasonable skill in the art that various modifications may be made without departing from the spirit and scope of the invention which is defined by the metes and bounds of the appended claims.

## Claims

1. A sleep analysis system, comprising:
a data generation unit configured to measure data for sleep analysis and including an illuminance measurement unit configured to selectively measure overall light or light of a specific wavelength;
a data processing unit configured to process the measured data and including a light amount calculation unit configured to selectively calculate an overall light amount of the light or a light amount of the specific wavelength based on measurement results of the illuminance measurement unit; and
a data output unit configured to display a circadian rhythm and a sleep state to the outside based on the processed data.

2. The sleep analysis system of claim 1, wherein the illuminance measurement unit comprises:
a lens through which light is incident;
a transmissive grating spaced apart from the lens by a predetermined distance and configured to disperse the incident light into visible light wavelengths; and
a camera sensor spaced apart from the transmissive grating by a predetermined distance d and configured to measure an intensity of light of the visible light wavelengths.

3. The sleep analysis system of claim 2, wherein the camera sensor is configured to:
measure an intensity of light over an entire visible light wavelength range; or
measure an intensity of light for each wavelength of the visible light by varying the distance d from the transmissive grating.

4. The sleep analysis system of claim 2, wherein the light amount calculation unit comprises:
a light amount measurement unit configured to obtain a light amount over an entire visible light wavelength range from an intensity of light measured by the camera sensor;
a pixel-based light amount collection unit configured to collect light amount information for each pixel unit of the camera sensor;
a wavelength conversion unit configured to convert pixel units into wavelength units;
a first light amount calculation unit configured to calculate a light amount over the entire visible light wavelength range from the light amount information converted into the wavelength units; and
a second light amount calculation unit configured to calculate a light amount at a specific wavelength from the light amount information converted into the wavelength units.

5. The sleep analysis system of claim 4, wherein the second light amount calculation unit is configured to calculate a light amount in a blue light wavelength range as the specific wavelength.

6. The sleep analysis system of claim 1, wherein the illuminance measurement unit comprises:
a light sensor configured to measure an overall intensity of light; or N light sensors (N being a natural number) each configured to measure an intensity of light of a specific wavelength,
wherein the light amount calculation unit is configured to:
obtain a light amount over an entire wavelength range of the light from the overall intensity of light; or obtain light amounts at each of N wavelengths.

7. The sleep analysis system of claim 1, wherein the data generation unit comprises:
an electrocardiogram measurement unit configured to measure a user's electrocardiogram;
a photoplethysmography measurement unit configured to measure a user's photoplethysmography in synchronization with the electrocardiogram measurement; and
a temperature measurement unit configured to measure a user's body temperature in synchronization with the electrocardiogram measurement.

8. The sleep analysis system of claim 7, wherein the data processing unit comprises:
a photoplethysmography calculation unit configured to derive oxygen saturation and blood pressure of the user from photoplethysmography measurement results;
an electrocardiogram calculation unit configured to derive a heart rate and heart rate variability of the user from electrocardiogram measurement results; and
a body temperature calculation unit configured to derive body temperature of the user from body temperature measurement results.

9. The sleep analysis system of claim 1, wherein the data output unit is configured to display information derived by the data processing unit to the outside according to time of a circadian cycle.

10. The sleep analysis system of claim 1, wherein the data output unit is configured to display a state of the user during sleep time among information derived by the data processing unit.

11. The sleep analysis system of claim 10, wherein the data output unit is configured to selectively display a change in overall daylight exposure during sleep time calculated by the light amount calculation unit and a change in a light amount of a specific wavelength.

12. The sleep analysis system of any one of claims 1 to 11, wherein the data generation unit, the data processing unit, and the data output unit are mounted in a wearable device.

13. A sleep analysis method comprising:
selectively measuring overall light or light of a specific wavelength;
selectively calculating an overall light amount or a light amount of the specific wavelength from the measured light;
displaying an alarm and measuring a user's electrocardiogram;
generating a synchronization signal synchronized with the electrocardiogram measurement;
measuring a user's photoplethysmography and a user's body temperature based on the synchronization signal;
calculating oxygen saturation, heart rate, blood pressure, heart rate variability, and body temperature of the user from the synchronized electrocardiogram, photoplethysmography, and body temperature measurements; and
displaying a circadian rhythm and a sleep state to the outside based on the calculated information.
